# EUROPÄISCHE PATENTSCHRIFT

(11) **EP 2 094 629 B1**
(45) Veröffentlichungstag und Bekanntmachung des Hinweises auf die Patenterteilung: **03.11.2010**
(21) Anmeldenummer: 07856421.8
(22) Anmeldetag: 06.12.2007
(51) Int. Cl.: C07C 7/11

(54) **VERFAHREN ZUR ENTFERNUNG VON CARBONYLVERBINDUNGEN AUS GASFÖRMIGEN KOHLENWASSERSTOFFEN**
METHOD FOR THE ELIMINATION OF CARBONYL COMPOUNDS FROM GASEOUS HYDROCARBONS
PROCÉDÉ POUR ÉLIMINER DES COMPOSÉS CARBONYLE D'HYDROCARBURES GAZEUX

(30) Priorität: 22.12.2006 DE 102006061076
(43) Veröffentlichungstag der Anmeldung: 02.09.2009
(73) Patentinhaber: Linde AG, 80331 München (DE); Basell Polyolefine GmbH, 65926 Frankfurt/Main (DE)
(72) Erfinder: MEISWINKEL, Andreas, 81479 München (DE); FREISINGER, Josef, 82049 Pullach (DE); KRAFT, Albert, 83607 Holzkirchen (DE); KIRZINGER, Anton, 81549 München (DE); DOMACHOWSKI, Tomasz, 51145 Köln (DE)
(86) Internationale Anmeldenummer: PCT/EP2007/010616
(87) Internationale Veröffentlichungsnummer: WO 2008/080500

(56) Entgegenhaltungen:
- EP-A- 0 264 280
- EP-A- 0 824 142

## Beschreibung

Die Erfindung betrifft ein Verfahren zur Behandlung eines hauptsächlich aus Kohlenwasserstoffen bestehenden Gases (Rohgas) in einer Anlage zur Herstellung von Kohlenwasserstoffen aus kohlenwasserstoffhaltigem Einsatz (Olefinanlage), wobei das Rohgas durch eine Wasserwäsche abgekühlt und in einem späteren Verfahrensschritt durch eine Laugenwäsche von unerwünschten Bestandteilen wie Kohlendioxid, Schwefelwasserstoff und Mercaptanen befreit wird.

In einer Anlage zur Herstellung von Kohlenwasserstoffen aus kohlenwasserstoffhaltigem Einsatz werden längerkettige Kohlenwasserstoffe zum Beispiel mittels Dampfspaltung in kürzerkettige Kohlenwasserstoffe umgewandelt. Das dabei entstehende gasförmige Gemisch aus kürzerkettigen Kohlenwasserstoffen (Rohgas) wird nach dem Stand der Technik mit Hilfe einer Wasserwäsche abgekühlt, verdichtet und in einer anschließenden Laugenwäsche von unerwünschten Bestandteilen wie Kohlendioxid, Schwefelwasserstoff und Mercaptanen befreit bevor es im Tieftemperaturzerlegungsteil der Anlage in die einzelnen Kohlenwasserstoffe getrennt wird.

Bei der Umwandlung der längerkettigen Kohlenwasserstoffe in kürzerkettige Kohlenwasserstoffe zum Beispiel mittels Dampfspaltung entstehen neben den kürzerkettigen Kohlenwasserstoffen auch Nebenprodukte wie sauerstoffhaltige organische Verbindungen, insbesondere Carbonylverbindungen und speziell Aldehyde wie zum Beispiel Acetaldehyd. Diese im Rohgas vorhandenen Carbonylverbindungen bilden in der Wasserwäsche sowie der Laugenwäsche und nachfolgenden Anlagenteilen Polymerisationsprodukte, welche zu Ablagerungen und daraus resultierenden Verlegungen der entsprechenden Anlagenteile führen. Insbesondere Acetaldehyd reagiert mit der Lauge über die Aldolreaktion/Aldolkondensation sehr stark zu Polymeren. Chemisch kann die Aldolkondensation durch die Zugabe von Chemikalien wie zum Beispiel Aminen oder Sulfiten gehemmt werden.

Nach dem Stand der Technik wird versucht, die Ablagerungen durch die Zugabe von Chemikalien wie Aminen oder Sulfiten unmittelbar in die Laugenwäsche zu minimieren (siehe zum Beispiel EP 0264280). Dies gelingt nur teilweise. Auch bei der Zugabe hoher Konzentrationen der Chemikalien gelangen genügend Carbonyle, insbesondere Aldehyde, in Kontakt mit der Lauge, so dass es zu einem kontinuierlichen Ablauf der Aldolkondensation, wenn auch in geringerem Maße, kommt. Die damit einhergehende kontinuierliche Ablagerung der Reaktionsprodukte macht eine regelmäßige und kostenintensive Reinigungsabschaltung der Anlage erforderlich.

Der vorliegenden Erfindung liegt daher die Aufgabe zugrunde, die Ablagerungen in der Laugenwäsche und die damit einhergehenden Reingungsabschaltungen weiter zu minimieren.

Die vorliegende Aufgabe wird verfahrensseitig erfindungsgemäß dadurch gelöst, dass das Rohgas vor der Laugenwäsche in eine separate Wäsche zur Entfernung von Carbonylverbindungen geführt wird, wobei als Waschmittel die wässrige Lösung einer Chemikalie eingesetzt wird, welche mit den im Rohgas befindlichen Carbonylverbindungen reagiert, wobei die dabei entstehenden Reaktionsprodukte in der Wasserphase verbleiben.

Der Grundgedanke der Erfindung besteht darin, die Carbonylverbindungen aus dem Rohgas zu entfernen, bevor das Rohgas und die darin enthaltenen Carbonylverbindungen in Kontakt mit Lauge kommen. Durch die erfindungsgemäße Entfernung der Carbonylverbindungen in einer separaten Wäsche vor der Laugenwäsche wird der Kontakt der Carbonylverbindungen mit der Lauge verhindert, so dass eine Aldolkondensation nicht ablaufen kann. Dadurch werden die aus der Aldolkondensation resultierenden Ablagerungen verhindert. Die Chemikalie, deren wässrige Lösung als Waschmittel verwendet wird, reagiert erfindungsgemäß mit den Carbonylverbindungen. Die bei dieser Reaktion gebildeten Reaktionsprodukte verbleiben in der Wasserphase, so dass die Carbonylverbindungen aus dem Rohgasstrom entfernt und in den Abwasserstrom überführt werden.

Vorteilhafterweise wird als Chemikalie eine Sulfit-, bevorzugt eine Bisulfitverbindung, besonders bevorzugt Natriumhydrogensulfit, eingesetzt. Durch die Wäsche des Rohgases mit der wässrigen Sulfit- bzw. Bisulfitlösung kommen die im Rohgas vorhandenen Carbonylverbindungen in Kontakt mit Sulfit oder Bisulfit und reagieren zu sehr gut wasserlöslichen Sulfit- bzw. Bisulfit-Addukten (siehe zum Beispiel S. Patai "The Chemistry of Carbonyl Group", Interscience Publishers, New York, 1966, 600-614). Daher sind Sulfit- bzw. Bisulfitverbindungen als Chemikalie für das erfindungsgemäße Verfahren bestens geeignet. Das besonders bevorzugt verwendete Natriumhydrogensulfit ist auch in großen Mengen günstig verfügbar. Zusätzlich lässt sich das Abwasser mit den gebildeten Sulfit- bzw. Bilsulfit-Addukten leicht aufbereiten.

In einer anderen Ausgestaltung der Erfindung werden als Chemikalie Amine, Hydroxylamine und/oder Hydrazine eingesetzt. Diese Chemikalien reagieren ebenfalls mit den Carbonylverbindungen zu gut wasserlöslichen Reaktionsprodukten, so dass die Carbonylverbindungen durch ihre wasserlöslichen Reaktionsprodukte effektiv aus dem Rohgas entfernt werden.

Zweckmäßigerweise wird der molare Quotient von Chemikalie zu Carbonylverbindungen zwischen 0.5 und 4, bevorzugt zwischen 1 und 1.5, eingestellt.

Durch die Einstellung des molaren Quotienten von Chemikalie zu Carbonylverbindungen kann die Wäsche an die anlagenspezifische Verunreinigung des Rohgases mit Carbonylverbindungen angepaßt werden, so dass nahezu alle Carbonylverbindungen bei optimalem Verbrauch der Chemikalie effektiv entfernt werden.

Als ebenso zweckmäßig erweist sich die Einstellung des Waschmittels als eine zwischen 1%ige bis 50%ige, bevorzugt 2%ige bis 10%ige, wässrige Lösung der Chemikalie. Dadurch kann die Einstellung des molaren Quotienten von Chemikalie zu Carbonylverbindung einfach vorgenommen werden.

Bevorzugt wird die Wäsche in einem Druckbereich zwischen 5 bar und 50 bar, besonders bevorzugt zwischen 20 bar und 35 bar, und bei einer Temperatur im Bereich zwischen 5°C und 90°C, besonders bevorzugt 10°C und 40°C, durchgeführt.

Zweckmäßigerweise wird die separate Wäsche in einem eigenen Behälter oder in einem separaten Teil der Laugenwäsche durchgeführt. Für die Durchführung des erfindungsgemäßen Verfahrens ist die Trennung der Wäsche zur Entfernung der Carbonylverbindungen von der Laugenwäsche essentiell. Dies lässt sich durch die Durchführung der Wäsche zur Entfernung der Carbonylverbindungen in einem eigenen Behälter oder in einem von der Lauge getrennten separaten Teil der Laugenwäsche erreichen. Dabei darf dieser separate Teil der Laugenwäsche keinen Kontakt zur Lauge oder Ablauge aufweisen.

Vorteilhafterweise werden die entstehenden Abwässer der Wäsche oxidativ aufgearbeitet. In einer bevorzugten Ausgestaltung der Erfindung werden die entstehenden Abwässer zusammen mit den Abwässern der Laugenwäsche oxidativ aufgearbeitet.

Zweckmäßigerweise erfolgt die oxidative Aufarbeitung der Abwässer durch ein Verfahren mittels Ozon und/oder Wasserstoffperoxyd Behandlung und/oder UV-Bestrählung.

Als ebenso zweckmäßig erweist sich die Einleitung der oxidativ aufgearbeiteten Abwässer in eine Kläranlage.

Mit der vorliegenden Erfindung gelingt es insbesondere, Carbonylverbindungen effektiv aus dem Rohgas zu entfernen und somit die durch die Aldolkondensation verursachten Ablagerungen in der Laugenwäsche deutlich zu minimieren.

## Patentansprüche

1. Verfahren zur Behandlung eines hauptsächlich aus Kohlenwasserstoffen bestehenden Gases in einer Anlage zur Herstellung von Kohlenwasserstoffen aus kohlenwasserstoffhaltigem Einsatz, wobei das Rohgas durch eine Wasserwäsche abgekühlt und in einem späteren Verfahrensschritt durch eine Laugenwäsche von unerwünschten Bestandteilen wie Kohlendioxid, Schwefelwasserstoff und Mercaptanen befreit wird, **dadurch gekennzeichnet, dass** das Rohgas vor der Laugenwäsche in eine separate Wäsche zur Entfernung von Carbonylverbindungen geführt wird, wobei als Waschmittel die wässrige Lösung einer Chemikalie eingesetzt wird, welche mit den im Rohgas befindlichen Carbonylverbindungen reagiert, wobei die dabei entstehenden Reaktionsprodukte in der Wasserphase verbleiben.

2. Verfahren nach Anspruch 1, **dadurch gekennzeichnet, dass** als Chemikalie eine Sulfitverbindung eingesetzt wird.

3. Verfahren nach Anspruch 1 oder 2, **dadurch gekennzeichnet, dass** als Chemikalie Amine, Hydroxylamine und/oder Hydrazine eingesetzt werden.

4. Verfahren nach einem der Ansprüche 1 bis 3, **dadurch gekennzeichnet, dass** der molare Quotient von Chemikalie zu Carbonylverbindungen zwischen 0.5 und 4 eingestellt wird.

5. Verfahren nach einem der Ansprüche 1 bis 4, **dadurch gekennzeichnet, dass** das Waschmittel als eine zwischen 1 %ige bis 50%ige wässrige Lösung der Chemikalie eingestellt wird.

6. Verfahren nach einem der Ansprüche 1 bis 5, **dadurch gekennzeichnet, dass** die Wäsche in einem Druckbereich zwischen 5 bar und 50 bar durchgeführt wird.

7. Verfahren nach einem der Ansprüche 1 bis 6, **dadurch gekennzeichnet, dass** die Wäsche bei einer Temperatur im Bereich zwischen 5°C und 90°C durchgeführt wird.

8. Verfahren nach einem der Ansprüche 1 bis 7, **dadurch gekennzeichnet, dass** die separate Wäsche in einem eigenen Behälter oder in einem separaten Teil der Laugenwäsche durchgeführt wird.

9. Verfahren nach einem der Ansprüche 1 bis 8, **dadurch gekennzeichnet, dass** die entstehenden Abwässer der Wäsche oxidativ aufgearbeitet werden.

10. Verfahren nach einem der Ansprüche 1 bis 9, **dadurch gekennzeichnet, dass** die entstehenden Abwässer zusammen mit den Abwässern der Laugenwäsche oxidativ aufgearbeitet werden.

11. Verfahren nach einem der Ansprüche 1 bis 10, **dadurch gekennzeichnet, dass** die oxidative Aufarbeitung der Abwässer durch ein Verfahren mittels Ozon und/oder Wasserstoffperoxyd Behandlung und/oder UV-Bestrahlung erfolgt.

12. Verfahren nach einem der Ansprüche 1 bis 11, **dadurch gekennzeichnet, dass** die oxidativ aufgearbeiteten Abwässer in eine Kläranlage eingeleitet werden.

## Claims

1. Process for the treatment of a gas comprising mainly hydrocarbons in a plant for preparing hydrocarbons from a hydrocarbon-containing feed, in which the raw gas is cooled by means of a water scrub and is freed of undesirable constituents such as carbon dioxide, hydrogen sulfide and mercaptans by means of an alkali scrub in a later process step, **characterized in that** the raw gas is introduced into a separate scrub for removing carbonyl compounds before the alkali scrub, with the aqueous solution of a chemical which reacts with the carbonyl compounds present in the raw gas being used as scrubbing medium and the reaction products formed remaining in the aqueous phase.

2. Process according to Claim 1, **characterized in that** a sulfite compound is used as chemical.

3. Process according to Claim 1 or 2, **characterized in that** amines, hydroxylamines and/or hydrazines are used as chemical.

4. Process according to any of Claims 1 to 3, **characterized in that** the molar ratio of chemical to carbonyl compounds is set in the range from 0.5 to 4.

5. Process according to any of Claims 1 to 4, **characterized in that** a from 1% strength to 50% strength aqueous solution of the chemical is used as scrubbing medium.

6. Process according to any of Claims 1 to 5, **characterized in that** the scrub is carried out in a pressure,range from 5 bar to 50 bar.

7. Process according to any of Claims 1 to 6, **characterized in that** the scrub is carried out at a temperature in the range from 5°C to 90°C.

8. Process according to any of Claims 1 to 7, **characterized in that** the separate scrub is carried out in a dedicated vessel or in a separate part of the alkali scrub.

9. Process according to any of Claims 1 to 8, **characterized in that** the wastewater formed in the scrub is worked up oxidatively.

10. Process according to any of Claims 1 to 9, **characterized in that** the wastewater formed is oxidatively worked up together with the wastewater from the alkali scrub.

11. Process according to any of claims 1 to 10, **characterized in that** the oxidative work-up of the wastewater is carried out by a process involving ozone and/or hydrogen peroxide treatment and/or UV irradiation.

12. Process according to any of Claims 1 to 11, **characterized in that** the oxidatively worked-up wastewater is introduced into a water treatment plant.

## Revendications

1. Procédé de traitement d'un gaz constitué principalement d'hydrocarbures dans une installation pour la fabrication d'hydrocarbures à partir d'une matière première contenant des hydrocarbures, le gaz brut étant refroidi par un lavage à l'eau et débarrassé des constituants indésirables tels que le dioxyde de carbone, le sulfure d'hydrogène et les mercaptans lors d'une étape de procédé ultérieure par un lavage alcalin, **caractérisé en ce que** le gaz brut est introduit dans un lavage distinct pour éliminer les composés de carbonyle avant le lavage alcalin, la solution aqueuse d'un produit chimique qui réagit avec les composés de carbonyle qui se trouvent dans le gaz brut étant utilisée en tant qu'agent de lavage, les produits de réaction alors formés restant dans la phase aqueuse.

2. Procédé selon la revendication 1, **caractérisé en ce qu'**un composé de sulfite est utilisé en tant que produit chimique.

3. Procédé selon la revendication 1 ou 2, **caractérisé en ce que** des amines, des hydroxylamines et/ou des hydrazines sont utilisées en tant que produit chimique.

4. Procédé selon l'une quelconque des revendications 1 à 3, **caractérisé en ce que** le quotient molaire entre le produit chimique et les composés de carbonyle est ajusté entre 0,5 et 4.

5. Procédé selon l'une quelconque des revendications 1 à 4, **caractérisé en ce que** l'agent de lavage est ajusté sous la forme d'une solution aqueuse de 1 % à 50 % du produit chimique.

6. Procédé selon l'une quelconque des revendications 1 à 5, **caractérisé en ce que** le lavage est réalisé dans une plage de pressions comprise entre 5 bar et 50 bar.

7. Procédé selon l'une quelconque des revendications 1 à 6, **caractérisé en ce que** le lavage est réalisé à une température dans la plage comprise entre 5 °C et 90 °C.

8. Procédé selon l'une quelconque des revendications 1 à 7, **caractérisé en ce que** le lavage distinct est réalisé dans un contenant propre ou dans une partie séparée du lavage alcalin.

9. Procédé selon l'une quelconque des revendications 1 à 8, **caractérisé en ce que** les eaux résiduaires formées par le lavage sont traitées par oxydation.

10. Procédé selon l'une quelconque des revendications 1 à 9, **caractérisé en ce que** les eaux résiduaires formées sont traitées par oxydation avec les eaux résiduaires du lavage alcalin.

11. Procédé selon l'une quelconque des revendications 1 à 10, **caractérisé en ce que** le traitement par oxydation des eaux résiduaires a lieu par un procédé au moyen d'ozone et/ou d'un traitement par du peroxyde d'hydrogène et/ou d'une exposition à un rayonnement UV.

12. Procédé selon l'une quelconque des revendications 1 à 11, **caractérisé en ce que** les eaux résiduaires traitées par oxydation sont introduites dans une station d'épuration.
